# EUROPEAN PATENT APPLICATION

(11) **EP 4 509 840 A1**
(43) Date of publication of application: **19.02.2025**
(21) Application number: 24189826.1
(22) Date of filing: 19.07.2024
(51) Int. Cl.: G01N 35/04, G01N 35/00

(54) **METHOD FOR MIXING IMMUNOASSAY REAGENT IN IMMUNOASSAY ANALYSIS DEVICE AND IMMUNOASSAY ANALYSIS DEVICE**

(30) Priority: 14.08.2023 CN 202311013030
(71) Applicant: Hunan Targeting Detection Technology Co., Ltd, Changsha City, Hunan Province (CN)
(72) Inventor: He, Feng, Changsha City (CN); Huang, Xin, Changsha City (CN); Li, Shicun, Changsha City (CN); Yin, Jun, Changsha City (CN)
(74) Representative: Zhu, Junyi

(57) **Abstract**

A method for mixing immunoassay reagent in immunoassay analysis device and an immunoassay analysis device are provided. The method disperses aggregated magnetic beads in a reagent tray of the immunoassay analysis device by keeping an immunoassay reagent tube containing the immunoassay reagent in a variable speed state during rotation and/or by exerting an impact force on the immunoassay reagent tube during rotation of the immunoassay reagent tube, so as to keep the immunoassay reagent in a mixed state. The method can mix the immunoassay reagent, avoid the phenomenon of magnetic beads' aggregation and ensure the accuracy of the testing results.

## Description

### Technical Field

The present invention relates to a method for mixing a reagent and a device using same, and particularly to a method for mixing an immunoassay reagent in an immunoassay analysis device and an immunoassay analysis device, which belong to the technical field of medical testing.

### BACKGROUND

It has been more than half a century since the first automatic chemical analysis instrument was manufactured, and the full automatic immunoassay analysis device has become technologically mature. The full automatic immunoassay analysis device has the advantages of high speed, high efficiency, high precision and repeated consistency, has been widely used in the fields of processing, production, testing and living assistance, and is bound to become a trend in the field of medical testing.

The reagent required for the reaction is stored in a kit, and the kit is mounted on a reagent tray, so the reagent tray of the full automatic immunoassay analysis device is the storage and supply unit of the reagent required for the reaction. When the reagent is to be aspirated, the kit on the reagent tray is rotated by means of rotation of the reagent tray to the position where a reagent needle needs to aspirate the reagent, and then the reagent in the kit is aspirated into an incubation tray by the reagent needle to react with the sample.

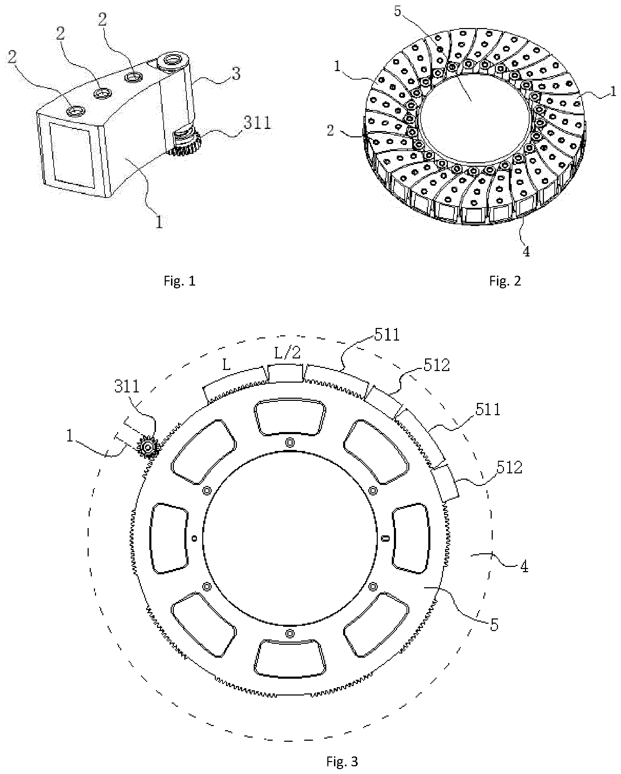

As shown in Fig. 1 and Fig. 2, a plurality of independent reagent chambers 2 are arranged in a kit 1, the upper end of each reagent chamber 2 is opened to facilitate the reagent needle to aspirate the reagent, and each reagent chamber 2 contains a different reagent; and one end of the kit 1 is also rotationally connected with an immunoassay reagent tube 3, the bottom of the immunoassay reagent tube 3 is provided with a reagent tube gear 311, a plurality of kits 1 are successively installed on a reagent turntable 4 along the circumferential direction of the reagent turntable 4, and the reagent turntable 4 can drive the plurality of kits 1 to rotate together. The immunoassay reagent tube 3 contains the immunoassay reagent, and the immunoassay reagent is prone to precipitation and accumulation due to the characteristics thereof and needs to keep rotating during the testing aspirating process to prevent precipitation and accumulation. Therefore, during the testing process, the prior art is that a toothed disc 5 is arranged in the central position of the reagent turntable 4, the reagent tube gear 311 at the bottom of the immunoassay reagent tube 3 in each kit is engaged with the toothed disc 5, the reagent turntable 4 is driven by a power mechanism to rotate, the toothed disc 5 in the central position is held still, and the plurality of kits 1 are driven by the reagent turntable 4 to rotate together, so as to drive the immunoassay reagent tubes 3 in the plurality of kits 1 on the reagent turntable 4 to keep rotating by engaged transmission of the toothed disc 5 and the reagent tube gear 311 to prevent the immunoassay reagent in the immunoassay reagent tubes 3 from precipitating and accumulating to ensure the accuracy of the testing results.

The prior art has the problem that although the immunoassay reagent tube is rotating most of the time, the mixing effect of the magnetic beads and the reagent solution in the immunoassay reagent is not very good, and the phenomenon of magnetic bead aggregation occurs, which affects the accuracy of the testing results.

### SUMMARY OF INVENTION

The primary technical problem to be solved by the present invention is to disclose a method for mixing an immunoassay reagent in an immunoassay analysis device and an immunoassay analysis device in view of the defects in the prior art, which can mix the immunoassay reagent, thus avoiding the phenomenon of magnetic bead aggregation and ensuring the accuracy of the testing results.

To solve the above technical problem, the present invention adopts the following technical solution: a method for mixing an immunoassay reagent in an immunoassay analysis device, which disperses aggregated magnetic beads in a reagent tray of an immunoassay analysis device by keeping an immunoassay reagent tube containing the immunoassay reagent in a variable speed state during rotation and/or by exerting an impact force on the immunoassay reagent tube during rotation of the immunoassay reagent tube, so as to keep the immunoassay reagent in a mixed state.

Preferably, continuous teeth distributed on a toothed disc in the reagent tray along a circumferential direction are designed into discontinuous teeth so that a plurality of tooth segments and notch segments are formed on the toothed disc, a kit is installed on a reagent turntable, the immunoassay reagent tube is rotationally connected to the kit, and the reagent turntable can rotate relative to the toothed disc, so as to drive a reagent tube gear at the bottom of the immunoassay reagent tube to move along the circumferential direction of the toothed disc.

In the working process, when the immunoassay reagent tube is located on the tooth segments on the toothed disc, the tooth segments are engaged with the reagent tube gear at the bottom of the immunoassay reagent tube to make the immunoassay reagent tube rotate; when the immunoassay reagent tube is located on the notch segments on the toothed disc, the immunoassay reagent tube loses power of rotation so that the rotation speed of the immunoassay reagent tube in the notch segments is changed once; and when the immunoassay reagent tube is located on the tooth segments on the toothed disc again, the tooth segments can be engaged with the reagent tube gear at the bottom of the immunoassay reagent tube again, and the speed of the immunoassay reagent tube is changed again to return to the rotation state, so repeatedly, until the rotation speed of the immunoassay reagent tube is repeatedly in a variable state in the working process.

When the immunoassay reagent tube is moved from the notch segments to the tooth segments on the toothed disc, the tooth segments that are just engaged with the reagent tube gear will form an impact force on the immunoassay reagent tube, which plays an impact role.

Preferably, the arc length of the tooth segments is set to L, and the arc length of the notch segments is L/2.

Preferably, the inside wall of the immunoassay reagent tube is provided with tube bumps; and the immunoassay reagent tube can further mix the immunoassay reagent by the tube bumps during rotation at a variable speed and under the action of the impact force.

Preferably, the number of the tube bumps is set to two, and the two tube bumps are distributed symmetrically about the central axis of the immunoassay reagent tube.

Preferably, in the working process, when it is not necessary to aspirate the reagent, the reagent turntable is held still by controlling the rotation of the toothed disc so that the immunoassay reagent tube rotates.

When it is necessary to aspirate various reagents in the kit, the reagent turntable is controlled to drive the kit to rotate to a reagent aspirating point A, the kit at point A is held still, and then various reagents in the kit are aspirated.

Preferably, the rotation direction of the toothed disc is controlled to be opposite to that of the reagent turntable.

The present invention also discloses an immunoassay analysis device, comprising a reagent tray and a cleaning tray arranged on one side of the reagent tray, wherein the reagent tray adopts the above method for mixing an immunoassay reagent to mix the immunoassay reagent.

Preferably, the cleaning tray comprises a cleaning tray barrel body, a turntable mechanism arranged in the cleaning tray barrel body and a needle body lifting mechanism arranged above the cleaning tray barrel body, injection needles and aspirating needles are arranged on the needle body lifting mechanism, reaction cups are placed on a turntable of the turntable mechanism, the reaction cups can be driven through the turntable to rotate, a cleaning tank is arranged in the cleaning tray barrel body and below the turntable, and the injection needles and the aspirating needles can be driven by the downward movement of the needle body lifting mechanism to move down to be inserted into the reaction cups to clean the reaction cups and to move down to be inserted into the cleaning tank to be cleaned after the reaction cups are removed.

Preferably, the cleaning tank comprises a fully enclosed annular tank body, and injection needle cleaning barrels and aspirating needle cleaning barrels which are arranged on the annular tank body, wherein the inner space of the injection needle cleaning barrels and the aspirating needle cleaning barrels is communicated with the inner space of the annular tank body, the bottom surface of the annular tank body is also provided with a drain pipe for draining water, and the top surfaces of each injection needle cleaning barrel and each aspirating needle cleaning barrel are respectively provided with openings for the injection needles and the aspirating needles to insert; and the injection needle cleaning barrels and the aspirating needle cleaning barrels are arranged according to the positions of the injection needles and the aspirating needles so that one injection needle is cleaned by one injection needle cleaning barrel and one aspirating needle is cleaned by one aspirating needle cleaning barrel.

Preferably, each aspirating needle cleaning barrel is vertically penetrated through the annular tank body, each aspirating needle cleaning barrel comprises an outer barrel body and an inner barrel body arranged in the outer barrel body, an annular space is formed between the outer barrel body and the inner barrel body, the annular space is communicated with the inner space of the annular tank body, the top surface of the inner barrel body is lower than that of the outer barrel body, the opening of each aspirating needle cleaning barrel is arranged on the top surface of the outer barrel body, the aspirating needle cleaning barrel arranged below the annular tank body is also provided with a water inlet, the water inlet is communicated with the bottom of the inner cavity of the inner barrel body, and the top of the inner cavity of the inner barrel body is communicated with the inner space of the outer barrel body.

Preferably, each injection needle cleaning barrel comprises a barrel body, the bottom end of the barrel body is arranged at the top of the annular tank body, the inner cavity of the barrel body is communicated with the inner space of the annular tank body, and the opening of each injection needle cleaning barrel is arranged on the top end of the barrel body.

Preferably, a reaction cup vibrating mechanism is arranged in the cleaning tray barrel body, the reaction cup vibrating mechanism comprises a guide rail I and a guide rail II which are arranged in the cleaning tray barrel body and an impactor which is slidably connected to the guide rail I and the guide rail II, an impactor driving mechanism is also arranged in the cleaning tray barrel body, and the impactor driving mechanism is used to drive a push block to move back and forth along the guide rail I and the guide rail II so as to use the impactor to repeatedly impact the reaction cups on the turntable located at an injection station so that the magnetic beads in the reaction cups are dispersed by the impact.

Preferably, the impactor driving mechanism comprises an impactor driving motor arranged in the cleaning tray barrel body and an impactor rotating shaft I rotationally connected in the cleaning tray barrel body, the output shaft of the impactor driving motor is in matched rotational connection with one end of the impactor rotating shaft I, the end surface of the other end of the impactor rotating shaft I is also provided with an impactor rotating shaft II, the central axis of the impactor rotating shaft I does not coincide with that of the impactor rotating shaft II, the impactor is provided with a slotted hole, the impactor rotating shaft II is inserted into the slotted hole for matched sliding connection so as to control the impactor driving motor to drive the impactor rotating shaft I and the impactor rotating shaft II to rotate, and the impactor can be driven by the contact between the impactor rotating shaft II and the inner circumferential surface of the slotted hole to move back and forth along the guide rail I and the guild rail II to repeatedly impact the reaction cups.

Preferably, three basic cleaning units are successively arranged on the needle body lifting mechanism along the circumferential direction of the turntable, the basic cleaning units comprise two basic cleaning units I and one basic cleaning unit II, an injection needle I and an aspirating needle are arranged in each basic cleaning unit I, an integrated double injection needle and an aspirating needle are arranged in the basic cleaning unit II, and the integrated double injection needle comprises an injection needle I and an injection needle II; and along the circumferential direction of the turntable, needle bodies are distributed on the needle body lifting mechanism in the following order: injection needle I, aspirating needle, injection needle I, aspirating needle, integrated double injection needle and aspirating needle.

Preferably, the integrated double injection needle is connected to a lifting plate of the needle body lifting mechanism through a limiting block, the limiting block comprises a base arranged on the lifting plate and a screw cap threaded to the base, the base comprises a bottom plate and a cylinder body arranged on the bottom plate, the cylinder body and the bottom plate are connected through a mounting through hole, the inner circumferential surface of the cylinder body penetrated by the mounting through hole is provided with a groove, the groove is concave along the radial direction of the cylinder body, one side of the groove is open, and the other three sides are closed.

The bottom of the screw cap is provided with a screw cap through hole, the integrated double injection needle is also provided with a guide block which is cylindrical, the diameter of the guide block is matched with that of the mounting through hole, and one side of the guide block is provided with a bump protruding radially; and during installation, the injection needle I and the injection needle II are successively penetrated through the screw cap through hole of the screw cap, the guide block and the mounting through hole of the base so that the bump of the guide block is stuck into the groove, the groove is matched with the bump to limit the injection needle I and the injection needle II in the circumferential direction, the screw cap is tightened onto the cylinder body, and the bump is pressed into the groove by the screw cap to limit the injection needle I and the injection needle II in the axial direction.

The present invention has the following beneficial effects: the reagent tube gear at the bottom of the immunoassay reagent tube can be driven to move along the circumferential direction of the toothed disc by designing the toothed disc to have discontinuous teeth and rotating the reagent turntable relative to the toothed disc, so as to realize the technical solution of dispersing aggregated magnetic beads by changing the state of uniform rotation during rotation of the immunoassay reagent tube and exerting an impact force during rotation of the immunoassay reagent tube. The reagent turntable is held still by controlling the rotation of the toothed disc so that the immunoassay reagent tube achieves the effect of active mixing. In this way, the entire testing time is greatly shortened compared with the previous mixing methods, thus improving the testing efficiency. By designing the cleaning tray in the immunoassay analysis device, the cleaning tank is integrated in the cleaning tray and located below the turntable, making full use of the inner space of the cleaning tray and the moving stroke of the needle body lifting mechanism. During normal operation of the cleaning tray, the needle body lifting mechanism is moved down so that the injection needles and the aspirating needles are moved down to the working station to clean the reaction cups. When the injection needles and the aspirating needles need to be cleaned, the reaction cups are removed, and then the needle body lifting mechanism continues to move down so that the injection needles and the aspirating needles are moved down beyond the working station until inserted into the cleaning tank to clean the needle bodies. Therefore, the present invention can not only ensure that the injection needles and the aspirating needles can normally clean the magnetic beads in the reaction cups, but also clean the injection needles and the aspirating needles, which simplifies the cleaning steps, improves the convenience of cleaning the injection needles and the aspirating needles and improves the cleaning effect of the injection needles and the aspirating needles. Through the design of the cleaning tank and the specific design of the cleaning steps, the inner and outer walls of the aspirating needles and the inner walls of the injection needles are cleaned at the same time, and the cleaning effect is improved. The reaction cup vibrating mechanism is arranged in the cleaning tray. When the injection needles inject solutions into the reaction cups, the reaction cup vibrating mechanism is used to repeatedly impact the reaction cups so that the magnetic beads in the reaction cups are dispersed by the repeated impact, and the magnetic beads are cleaned with the cleaning solutions injected by the injection needles, thus ensuring the cleaning effect. The reaction cup vibrating mechanism is arranged in the cleaning tray. When the injection needles inject solutions into the reaction cups, the reaction cup vibrating mechanism is used to repeatedly impact the reaction cups so that the magnetic beads in the reaction cups are dispersed by the repeated impact, and the magnetic beads are cleaned with the cleaning solutions injected by the injection needles, thus ensuring the cleaning effect. With a double injection needle mechanism, the present invention can use two different cleaning solutions to cooperate with each other, which can not only achieve the effect of cleaning the magnetic beads in the reaction cups, but also remove the residual bubbles in the reaction cups, thus ensuring the accuracy of the final testing results.

### BRIEF DESCRIPTION OF DRAWINGS

Fig. 1 is a three-dimensional structural schematic diagram of a kit;
Fig. 2 is a three-dimensional structural schematic diagram of a matching position relationship between a reagent turntable and a toothed disc.
Fig. 3 is a structural schematic diagram of a method for mixing an immunoassay reagent in embodiments of the present invention.
Fig. 4 is a local three-dimensional structural schematic diagram of the interior of an immunoassay reagent tube in embodiments of the present invention.
Fig. 5 is a structural schematic diagram of aspirating a reagent in the prior art.
Fig. 6 is a structural schematic diagram of aspirating a reagent in embodiments of the present invention.
Fig. 7 is a three-dimensional structural schematic diagram of a reagent tray in embodiments of the present invention.
Fig. 8 is an axial sectional structural schematic diagram of a reagent tray in embodiments of the present invention.
Fig. 9 is a local amplified structural schematic diagram of part B in Fig. 8.
Fig. 10 is a top structural schematic diagram of an immunoassay analysis device in embodiments of the present invention.
Fig. 11 is a three-dimensional structural schematic diagram of a cleaning tray in embodiments of the present invention.
Fig. 12 is an axial sectional structural schematic diagram of a cleaning tray in embodiments of the present invention.
Fig. 13 is a top structural schematic diagram of a cleaning tray in embodiments of the present invention.
Fig. 14 is a top structural schematic diagram of a cleaning tank in embodiments of the present invention.
Fig. 15 is a three-dimensional structural schematic diagram of a cleaning tank in embodiments of the present invention.
Fig. 16 is a schematic diagram of inserting an injection needle into a reaction cup to inject a cleaning solution.
Fig. 17 is a schematic diagram of inserting an aspirating needle into a reaction cup to aspirate a solution.
Fig. 18 is a sectional structural schematic diagram along C-C line in Fig. 14.
Fig. 19 is a schematic diagram of cleaning an aspirating needle in Fig. 18.
Fig. 20 is a sectional structural schematic diagram along D-D line in Fig. 14.
Fig. 21 is a schematic diagram of cleaning an injection needle in Fig. 20.
Fig. 22 is a local sectional structural schematic diagram of a turntable in Fig. 12.
Fig. 23 is a three-dimensional structural schematic diagram of a turntable mechanism in embodiments of the present invention.
Fig. 24 is a main structural schematic diagram of a turntable mechanism in embodiments of the present invention.
Fig. 25 is a schematic diagram of distribution positions of an injection station, an aspirating station and a reaction cup vibrating mechanism in embodiments of the present invention.
Fig. 26 is a schematic diagram of magnetic field position distribution in embodiments of the present invention.
Fig. 27 is a local three-dimensional structural schematic diagram of a cleaning tray from which a needle body lifting mechanism and a turntable mechanism are removed in embodiments of the present invention.
Fig. 28 is a local three-dimensional structural schematic diagram of a reaction cup vibrating mechanism in Fig. 27.
Fig. 29 is a local three-dimensional structural schematic diagram of a reaction cup vibrating mechanism from which an impactor is removed in Fig. 28.
Fig. 30 is a local three-dimensional structural schematic diagram of an impactor rotating shaft I and an impactor rotating shaft II in Fig. 29.
Fig. 31 is a local axial sectional structural schematic diagram of a reaction cup arranged in a cup hole of a turntable in embodiments of the present invention.
Fig. 32 is a local sectional structural schematic diagram of a needle body lifting mechanism in Fig. 12.
Fig. 33 is a local three-dimensional structural schematic diagram of a needle body lifting mechanism in Fig. 12.
Fig. 34 is a local three-dimensional structural schematic diagram of an integrated double injection needle in Fig. 33.
Fig. 35 is a three-dimensional structural schematic diagram of a base of a limiting block in a cleaning tray in embodiments of the present invention.
Fig. 36 is a three-dimensional structural schematic diagram of an integrated double injection needle penetrating a screw cap and a guide block in embodiments of the present invention.
Fig. 37 is a three-dimensional structural schematic diagram of an integrated double injection needle installed on a lifting plate in embodiments of the present invention.

In the figures: 1. kit, 2. reagent chamber, 3. immunoassay reagent tube, 311. reagent tube gear, 312. tube bump, 4. reagent turntable, 5. toothed disc, 511. tooth segment, 512. notch segment, 6. reagent tray, 611. reagent tray barrel body, 612. reagent through hole, 7. driving motor I, 8. caster, 9. driving motor II, 10. lug boss, 11. toothed disc bearing, 12. driving toothed disc I, 13. driving toothed disc II, 14. sample transfer channel, 15. incubation tray, 16. cleaning tray, 17. optical testing mechanism, 18. consumables warehouse, 181. test chip module, 182. tip module, 183. reaction cup module, 19. cup grabbing swing arm, 20. reagent needle swing arm, 21. reagent needle cleaning pool, 22. cleaning tray barrel body, 221. cleaning tray partition plate, 222. upper barrel body space, 223. lower barrel body space, 23. turntable mechanism, 231. turntable, 232. turntable motor and reducer, 2321. rotating shaft, 24. needle body lifting mechanism, 241. lifting plate, 242. lifting motor, 25. reaction cup, 251. cup body, 252. cup body flange, 26. cleaning tank, 261. annular tank body, 2611. inner space, 262. injection needle cleaning barrel, 2621. barrel body, 263. aspirating needle cleaning barrel, 2631. outer barrel body, 2632. inner barrel body, 26321.inner cavity, 2633. annular space, 264. drain pipe, 27. injection needle, 271.injection needle I, 272. injection needle II, 28. aspirating needle, 29. opening, 30. solution, 31. water inlet, 32. cup hole, 33. reaction cup through hole, 34. sensor I, 35. induction ring, 351. induction channel, 36. sensor II, 37. basic cleaning unit I, 38. basic cleaning unit II, 39. magnet retaining ring, 40. magnetic field, 41. reaction cup vibrating mechanism, 411. guide rail I, 412. guide rail II, 413. impactor, 4131. arc end, 42. impactor rotating shaft I, 43. impactor rotating shaft II, 431. impact bearing, 432. lock bolt, 44. slotted hole, 45. induction disk, 46. sensor III, 47. impact gap, 48. guide sleeve, 49. guide rod, 50. lead screw, 51. nut sleeve, 52. upper position sensor, 53. lower position sensor, 54. support rod, 55. induction sheet, 56. base, 561. bottom plate, 562. cylinder body, 563. mounting through hole, 57. screw cap, 571. screw cap through hole, 58. groove, 59. guide block, 591. bump, 60. three-dimensional movable robotic arm.

### DESCRIPTION OF EMBODIMENTS

The technical solution of the present invention is further described in detail below in combination with the drawings and specific embodiments.

Although the immunoassay reagent tube is rotated most of the time, the mixing effect of the magnetic beads and the reagent solution in the immunoassay reagent is not very good, and the phenomenon of magnetic bead aggregation occurs. The applicant finds through research that the main reason is that the immunoassay reagent tube is rotated most of the time, but is always in a state of slower uniform rotation, and the magnetic beads are very easy to be thrown to the inside wall of the immunoassay reagent tube under the action of a centrifugal force, resulting in the phenomenon of magnetic bead aggregation.

The present invention discloses a method for mixing an immunoassay reagent in an immunoassay analysis device, which disperses aggregated magnetic beads by keeping an immunoassay reagent tube in a variable speed state during rotation and/or by exerting an impact force on the immunoassay reagent tube during rotation of the immunoassay reagent tube, so as to keep the immunoassay reagent in a mixed state. The applicant finds through research that the above problem can be solved by changing the state of uniform rotation during rotation of the immunoassay reagent tube, i.e., the immunoassay reagent tube is not kept in a state of uniform rotation, and the rotation speed is changed so that the centrifugal force on the immunoassay reagent tube will also be changed, thus changing the state of magnetic bead aggregation; or the aggregated magnetic beads can be dispersed by exerting an external impact force during rotation of the immunoassay reagent tube. Most preferentially, the phenomenon of magnetic bead aggregation is eliminated by the above two technical solutions. The applicant finds through tests that such technical solutions can achieve the best effect, and no phenomenon of magnetic bead aggregation occurs in the immunoassay reagent simultaneously mixed by the two technical solutions.

Embodiment: the technical solution of dispersing aggregated magnetic beads by changing the state of uniform rotation during rotation of the immunoassay reagent tube and exerting an impact force during rotation of the immunoassay reagent tube is shown in Fig. 3, and the applicant designs continuous teeth distributed on the toothed disc 5 along a circumferential direction into discontinuous teeth to form notches between the adjacent tooth segments. In the present embodiment, the toothed disc 5 is provided with ten tooth segments 511 and ten notch segments 512. In the working process, when the immunoassay reagent tube is located on the tooth segments 511 on the toothed disc 5, the tooth segments 511 are engaged with the reagent tube gear 311 at the bottom of the immunoassay reagent tube to provide a rotational force to the immunoassay reagent tube to make the immunoassay reagent tube rotate; when the immunoassay reagent tube is located on the notch segments 512 on the toothed disc 5, since no teeth in the notch segments 512 are engaged with the reagent tube gear 311, the immunoassay reagent tube loses power of rotation, and since the rotation speed is not high, the immunoassay reagent tube slows down and sometimes even stops rotating after losing power of rotation, so the rotation speed of the immunoassay reagent tube in the notch segments 512 is changed once; and when the immunoassay reagent tube is located on the tooth segments 511 on the toothed disc 5 again, the tooth segments 511 can be engaged with the reagent tube gear 311 at the bottom of the immunoassay reagent tube again, a rotational force is provided to the immunoassay reagent tube, and the speed of the immunoassay reagent tube is changed again to return to the rotation state after obtaining the force, so repeatedly, until the rotation speed of the immunoassay reagent tube is repeatedly in a variable state in the working process. In addition, when the immunoassay reagent tube is moved from the notch segments 512 to the tooth segments 511 on the toothed disc, the tooth segments 511 that are just engaged with the reagent tube gear 311 will form an impact force on the immunoassay reagent tube and play an impact role. The immunoassay reagent tube disperses the aggregated magnetic beads therein under the double actions of the above rotational variable speed state and the impact force, so as to keep the immunoassay reagent in a mixed state. The present embodiment can mix the immunoassay reagent, thus avoiding the phenomenon of magnetic bead aggregation and ensuring the accuracy of the testing results.

The applicant finds through many tests that the arc length of the tooth segments 511 is set to L and the arc length of the notch segments 512 is L/2, which can achieve the best mixing effect of the immunoassay reagent in the immunoassay reagent tube.

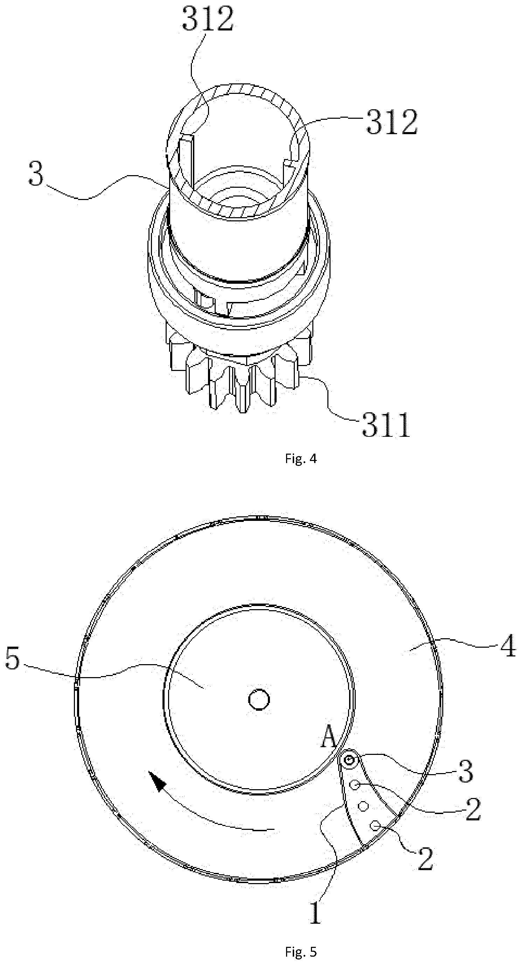

To further improve the mixing effect, the inside wall of the immunoassay reagent tube 3 is provided with tube bumps 312, as shown in Fig. 4. The immunoassay reagent tube 3 can further mix the immunoassay reagent by the tube bumps 312 during rotation at a variable speed and under the action of the impact force, which can further improve the mixing effect. In the present embodiment, the number of the tube bumps 312 is set to two, and the two tube bumps 312 are distributed symmetrically about the central axis of the immunoassay reagent tube 3.

The prior art has another problem. As shown in Fig. 5, in the prior art, the toothed disc 5 in the central position is held still in the working process, and the reagent turntable 4 is controlled to drive the kit 1 to rotate so that the immunoassay reagent tube 3 on the kit 1 rotates through engagement of the toothed disc 5 and the reagent tube gear 311. Generally, reagents contained in the immunoassay reagent tube 3 and a plurality of reagent chambers 2 on the kit 1 are used for testing of the same index. When an index is tested, a reagent aspirating point is assumed to be point A. When the immunoassay reagent in the immunoassay reagent tube 3 on the kit 1 is to be aspirated, the reagent turntable 4 is controlled first to drive the kit 1 to rotate to point A, and then the immunoassay reagent in the immunoassay reagent tube 3 is aspirated. After aspiration, the kit 1 shall not be stopped here, but the reagent turntable 4 shall be controlled to drive the kit 1 to continue to turn a circle to reach point A again, and then the reagent contained in one reagent chamber 2 on the kit 1 is aspirated. After aspiration, the reagent turntable 4 is controlled to drive the kit 1 to continue to turn a circle to reach point A again, and the reagent contained in the next reagent chamber 2 on the kit 1 is aspirated, so repeatedly, until the reagents contained in the immunoassay reagent tube 3 and the plurality of reagent chambers 2 on the kit 1 are aspirated, thereby completing the test of the index. Such mixing method will lead to an increase in the entire testing time, thus reducing the testing efficiency.

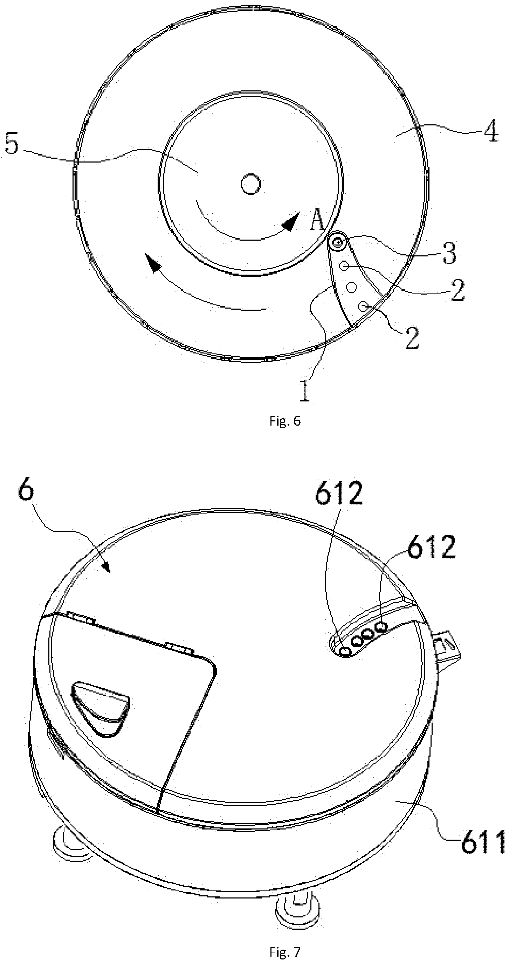

Therefore, as shown in Fig. 6, the applicant arranges a driving mechanism I and a driving mechanism II on the toothed disc 5 and the reagent turntable 4 respectively. In the working process, the toothed disc 5 is driven by the driving mechanism I to rotate, and the reagent turntable 4 is held still so that the kit 1 on the reagent turntable 4 is also stationary, and the immunoassay reagent tube 3 on the kit 1 rotates only by engagement of the toothed disc 5 and the reagent tube gear 311. When it is necessary to aspirate various reagents in the kit 1, the reagent turntable 4 is driven by the driving mechanism II to drive the kit 1 to rotate to the reagent aspirating point A, and then the kit 1 at point A is held still. At this time, the toothed disc 5 is still rotated, so the immunoassay reagent tube 3 on the kit 1 is still in a state of rotation. Therefore, after the immunoassay reagent in the immunoassay reagent tube 3 on the kit 1 is aspirated, the kit 1 can still be fixed at point A, so as to continue to aspirate reagents in several other reagent chambers 2 on the kit 1. In this way, compared with the previous mixing methods, the entire testing time is greatly shortened, thus improving the testing efficiency.

In the present embodiment, the rotation direction of the toothed disc 5 is controlled to be opposite to that of the reagent turntable 4 so that the toothed disc 5 can be in a state of continuous rotation, and the reagent turntable 4, whether rotating or not, can cause the immunoassay reagent tube 3 of the kit 1 on the reagent turntable 4 to keep rotating, avoiding precipitation and accumulation of the immunoassay reagent in the immunoassay reagent tube 3.

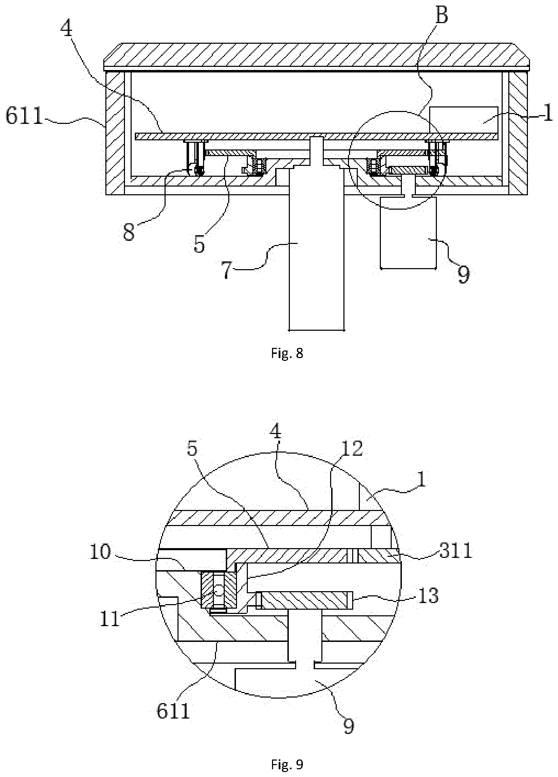

As shown in Fig. 7 and Fig. 8, the reagent turntable 4 is installed in the reagent tray 6 of the immunoassay analysis device, the reagent tray 6 comprises a reagent tray barrel body 611 and a driving mechanism I arranged at the bottom of the reagent tray barrel body 611, the reagent turntable 4 is located in the reagent tray barrel body 611, the driving mechanism I is a driving motor I 7, the output shaft of the driving motor I 7 is connected with the reagent turntable 4, and the reagent turntable 4 can be driven by the action of the driving motor 17 to rotate; and the bottom of the reagent turntable 4 is also provided with casters 8, and the casters 8 are in contact with the inner bottom of the reagent tray barrel body 611, so as to ensure that the reagent turntable 4 rotates more smoothly. The toothed disc 5 is rotationally connected in the reagent tray barrel body 611 and below the reagent turntable 4, the toothed disc 5 and the reagent turntable 4 are coaxial, the bottom of the reagent tray barrel body 611 is also provided with a driving mechanism II, the driving mechanism II is a driving motor II 9, the output shaft of the driving motor II 9 is in matched transmission connection with the toothed disc 5, and the toothed disc 5 can be driven by the action of the driving motor II 9 to rotate. The top of the reagent tray barrel body 611 is provided with a plurality of reagent through holes 612 for aspirating reagents, and the position of the reagent through holes 612 is the reagent aspirating point A.

As shown in Fig. 9, the inner bottom of the reagent tray barrel body 611 is provided with a lug boss 10, the inner ring of the toothed disc bearing 11 is sleeved on the outer circumferential surface of the lug boss 10, the toothed disc 5 is connected with the outer ring of the toothed disc bearing 11, the outer ring of the toothed disc bearing 11 is sleeved with a driving toothed disc I 12, the output shaft of the driving motor II 9 is also provided with a driving toothed disc II 13, and the driving toothed disc 112 is in engaged transmission connection with the driving toothed disc II 13 so that the toothed disc 5 can be driven under the action of the driving motor II 9 to rotate. The reagent tube gear 311 of the immunoassay reagent tube on the kit 1 installed on the reagent turntable 4 is in engaged transmission connection with the toothed disc 5.

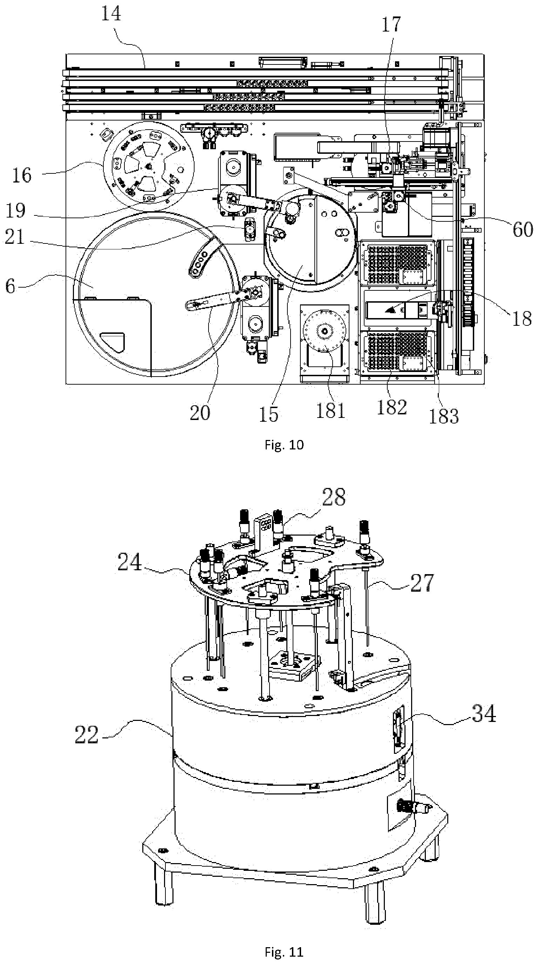

As shown in Fig. 10, the present invention also discloses an immunoassay analysis device, comprising a sample transfer channel 14, and the reagent tray 6, the incubation tray 15, the cleaning tray 16, an optical testing mechanism 17 and a consumables warehouse 18 which are arranged on one side of the sample transfer channel 14, a cup grabbing swing arm 19 is also arranged between the incubation tray 15 and the cleaning tray 16, the cup grabbing swing arm 19 is used for switching and grabbing the reaction cup back and forth between the incubation tray 15 and the cleaning tray 16, a reagent needle swing arm 20 is also arranged between the incubation tray 15 and the reagent tray 6, the reagent needle swing arm 20 is used for aspirating and adding the reagent from the reagent tray 6 into the reaction cup of the incubation tray 15, and a reagent needle cleaning pool 21 is arranged beside the reagent needle swing arm 20 to clean the reagent needle swing arm 20 after the reagent needle swing arm 20 aspirates a reagent so that the reagent needle swing arm 20 aspirates another reagent. A test chip module 181, a tip module 182 and a reaction cup module 183 are arranged in the consumables warehouse 18, and a plurality of components are stored in each module, i.e., a plurality of test chips are placed in the test chip module 181, a plurality of tips are placed in the tip module 182, and a plurality of reaction cups are placed in the reaction cup module 183 so that the immunoassay analysis device can perform multiple tests. A three-dimensional movable robotic arm 60 is also arranged among the incubation tray 15, the optical testing mechanism 17 and the consumables warehouse 18, and the three-dimensional movable robotic arm 60 is used for replacing consumables and aspirating samples to be tested in the reaction cup of the incubation tray 15 to the optical testing mechanism 17 for testing.

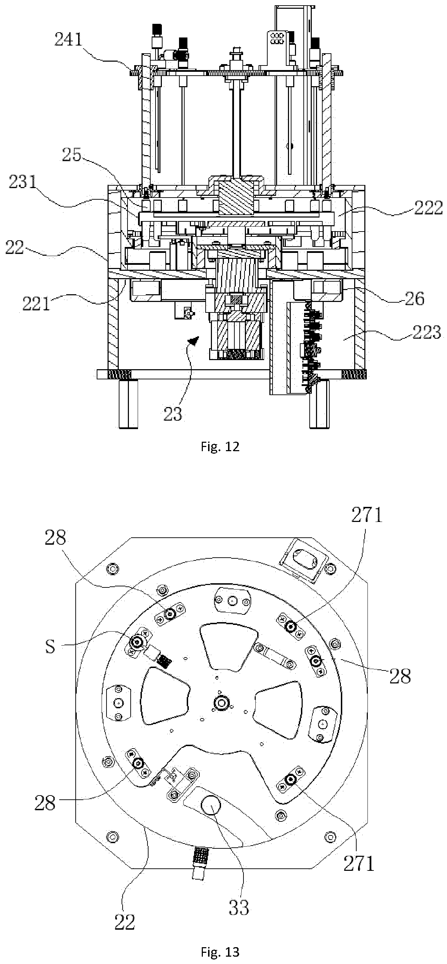

As shown in Fig. 11 and Fig. 12, the cleaning tray comprises a cleaning tray barrel body 22, a turntable mechanism 23 arranged in the cleaning tray barrel body 22 and a needle body lifting mechanism 24 arranged above the cleaning tray barrel body 22; and the reaction cup 25 is placed on a turntable 231 of the turntable mechanism 23, and the reaction cup 25 can be driven by the turntable 231 to rotate. A cleaning tank 26 is arranged in the cleaning tray barrel body 22 and below the turntable 231, and the needle body lifting mechanism 24 is provided with injection needles 27 and aspirating needles 28. The method for cleaning the injection needles and the aspirating needles in the cleaning tray of the immunoassay analysis device is that during normal operation, the injection needles and the aspirating needles can be driven by the downward movement of the needle body lifting mechanism 24 to move down to be inserted into the reaction cups to clean the reaction cups, when the injection needles and the aspirating needles need to be cleaned, the reaction cups 25 on the turntable 231 are removed first, and the downward movement of the needle body lifting mechanism 24 is controlled so that the injection needles and the aspirating needles are inserted into the cleaning tank 26 by penetrating the turntable for cleaning. In the present embodiment, the cleaning tank is integrated in the cleaning tray and located below the turntable, making full use of the inner space of the cleaning tray and the moving stroke of the needle body lifting mechanism. During normal operation of the cleaning tray, the needle body lifting mechanism is moved down so that the injection needles and the aspirating needles are moved down to the working station to clean the reaction cups. When the injection needles and the aspirating needles need to be cleaned, the reaction cups are removed, and then the needle body lifting mechanism continues to move down so that the injection needles and the aspirating needles are moved down beyond the working station until inserted into the cleaning tank to clean the needle bodies. Therefore, the present embodiment can not only ensure that the injection needles and the aspirating needles can normally clean the magnetic beads in the reaction cups, but also clean the injection needles and the aspirating needles, which simplifies the cleaning steps, improves the convenience of cleaning the injection needles and the aspirating needles and improves the cleaning effect of the injection needles and the aspirating needles.

As shown in Fig. 13, two basic cleaning units I and one basic cleaning unit II are successively arranged on the needle body lifting mechanism 24 along the circumferential direction of the turntable 231, an injection station and an aspirating station are arranged in each basic cleaning unit, an injection needle 1271 and an aspirating needle 28 are respectively arranged at the injection station and the aspirating station of each basic cleaning unit I, an integrated double injection needle S and an aspirating needle 28 are respectively arranged at the injection station and the aspirating station of the basic cleaning unit II, and the integrated double injection needle S comprises an injection needle I 271 and an injection needle II 272. Along the circumferential direction of the turntable 231, the needle bodies are distributed on the needle body lifting mechanism 24 in the following order: injection needle 1271, aspirating needle 28, injection needle I 271, aspirating needle 28, integrated double injection needle S and aspirating needle 28.

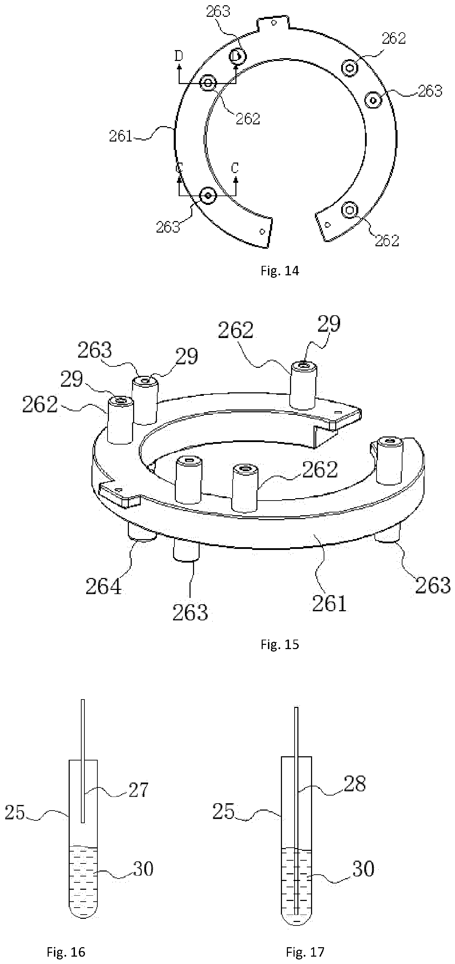

Since the injection needles and the aspirating needles are distributed along the circumferential direction of the turntable, as shown in Fig. 14 and Fig. 15, the cleaning tank 26 is also set to have an annular shape, the cleaning tank 26 comprises a fully enclosed annular tank body 261, and injection needle cleaning barrels 262 and aspirating needle cleaning barrels 263 which are arranged on the annular tank body 261, wherein the inner space of the injection needle cleaning barrels 912 and the inner space of the aspirating needle cleaning barrels 263 are communicated with the inner space of the annular tank body 261, the bottom surface of the annular tank body 261 is also provided with a drain pipe 264 for draining water, and the top surfaces of each injection needle cleaning barrel 912 and each aspirating needle cleaning barrel 263 are respectively provided with openings 29 for the injection needles and the aspirating needles to insert. The injection needle cleaning barrels 262 and the aspirating needle cleaning barrels 263 are arranged according to the distribution positions of the needle bodies on the needle body lifting mechanism 24 so that one injection needle is cleaned by one injection needle cleaning barrel 262 and one aspirating needle is cleaned by one aspirating needle cleaning barrel 263. Therefore, the order of the cleaning barrels on the annular tank body in the present embodiment is as follows: injection needle cleaning barrel 262, aspirating needle cleaning barrel 263, injection needle cleaning barrel 262, aspirating needle cleaning barrel 263, injection needle cleaning barrel 262 and aspirating needle cleaning barrel 263. With the design of the annular tank body and the annularly distributed cleaning barrels, the present embodiment can clean the injection needles and the aspirating needles at the same time, which further improves the cleaning efficiency.

To further improve the cleaning effect, the applicant finds through research that as shown in Fig. 16 and Fig. 17, in daily work, in the process of cleaning the magnetic beads in the reaction cups 25 on the cleaning tray, when the injection needles 27 are used to inject a solution into the reaction cups 25, the injection needles 27 are inserted into the solution 30 in the reaction cups 25, so it is only necessary to clean the inner walls of the injection needles 27 when cleaning the injection needles 27; and when the aspirating needles 28 are used to aspirate the solution from the reaction cups 25, the aspirating needles 28 shall be inserted into the solution 30 in the reaction cups 25, so it is necessary to clean the inner walls and the outer walls of the aspirating needles 28 when cleaning the aspirating needles 28.

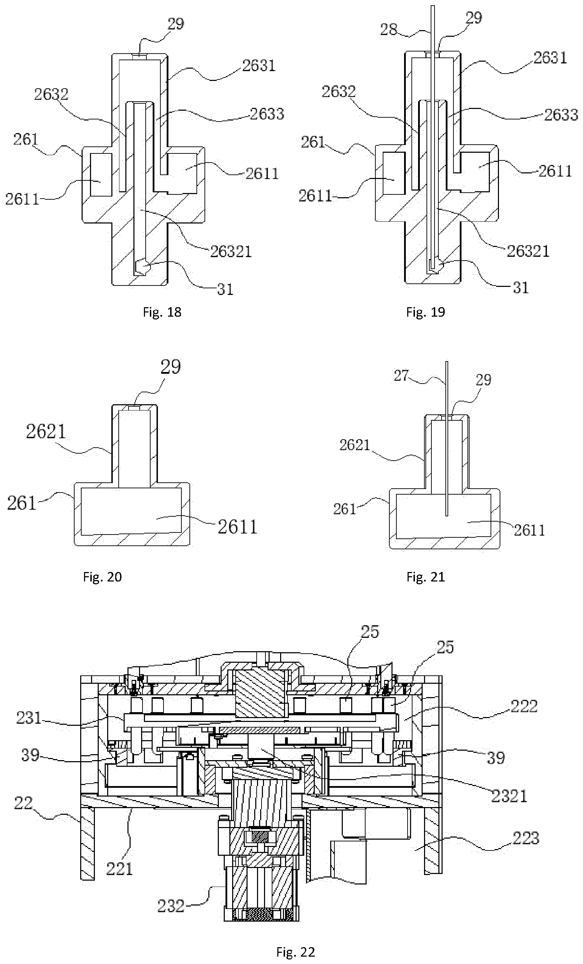

Therefore, as shown in Fig. 18 and Fig. 19, each aspirating needle cleaning barrel 263 is vertically penetrated through the annular tank body 261, each aspirating needle cleaning barrel 263 comprises an outer barrel body 2631 and an inner barrel body 2632 arranged in the outer barrel body 2631, an annular space 2633 is formed between the outer barrel body 2631 and the inner barrel body 2632, the annular space 2633 is communicated with the inner space 2611 of the annular tank body 261, the top surface of the inner barrel body 2632 is lower than that of the outer barrel body 2631, the opening 29 of the aspirating needle cleaning barrel 263 is arranged on the top surface of the outer barrel body 2631, the aspirating needle cleaning barrel 263 arranged below the annular tank body 261 is also provided with a water inlet 31, the water inlet 31 is communicated with the bottom of the inner cavity 26321 of the inner barrel body 2632, and the top of the inner cavity 26321 of the inner barrel body 2632 is communicated with the inner space of the outer barrel body 2631.

When the aspirating needles 28 are cleaned, the aspirating needles 28 are controlled to be inserted into the inner cavity 26321 of the inner barrel body 2632 through the opening 29 at the top of the aspirating needle cleaning barrel 263, an external water pump is used to transfer cleaning water to the inner cavity 26321 of the inner barrel body 2632 from the water inlet 31, and the aspirating needles 28 are controlled to start inspiration so that a part of cleaning water is aspirated by the aspirating needles 28, so as to clean the inner walls of the aspirating needles 28 with the cleaning water. Meanwhile, since the water supply of the water pump is greater than the water absorption of the aspirating needles 28, most of the cleaning water is finally drained through the drain pipe 264 after flowing through the inner cavity 26321 of the inner barrel body 2632, the annular space 2633 and the inner space 2611 of the annular tank body 261 so that the cleaning water flowing through the inner cavity 26321 of the inner barrel body 2632 is used to clean the outer walls of the aspirating needles 28. With the above method, the inner walls and the outer walls of the aspirating needles 28 are cleaned simultaneously, thus improving the cleaning effect.

The water inlet 31 is arranged on the side of the aspirating needle cleaning barrel 263 so that when the aspirating needles are cleaned, the cleaning water enters the inner cavity 26321 of the inner barrel body 2632 from the side positions of the aspirating needles 28. In this way, the cleaning water is not directly flushed into the aspirating needles from the bottoms of the aspirating needles, but is aspirated into the aspirating needles, ensuring the cleaning effect.

As shown in Fig. 20 and Fig. 21, each injection needle cleaning barrel 262 comprises a barrel body 2621, the bottom end of the barrel body 2621 is arranged at the top of the annular tank body 261, the inner cavity of the barrel body 2621 is communicated with the inner space 2611 of the annular tank body 261, and the opening 29 of the injection needle cleaning barrel 262 is arranged on the top end of the barrel body 2621.

When the injection needles 27 are cleaned, the injection needles 27 are controlled to be inserted into the inner cavity of the barrel body 2621 through the opening 29 at the top of the barrel body 2621, cleaning water is injected by the injection needles 27, and the cleaning water is injected into the inner space 2611 of the annular tank body 261 and finally drained through the drain pipe 264, so as to clean the inner walls of the injection needles 27 with the cleaning water flowing through the injection needles 27, improving the cleaning effect.

As shown in Fig. 12, a cleaning tray partition plate 221 is arranged in the cleaning tray barrel body 22, and the interior of the cleaning tray barrel body 22 is divided into an upper barrel body space 222 located in an upper position and a lower barrel body space 223 located in a lower position by the cleaning tray partition plate 221. The turntable 231 of the turntable mechanism 23 is located in the upper barrel body space 222, an insulation material and a heating device (not shown in the figure) are arranged in the upper barrel body space 222, and the heating device is an electric heating film. The interior of the upper barrel body space 222 is heated and maintained at a certain temperature by the insulation material and the heating device, because the reaction cups need to be maintained at a certain temperature after being placed on the turntable. The present embodiment can ensure that the reaction cups on the turntable are always in an environment with a certain operating temperature by arranging the turntable in the heat preservation space of the upper barrel body space.

As shown in Fig. 13, Fig. 22 and Fig. 23, the turntable mechanism 23 also comprises a turntable motor and a reducer 232, the turntable motor and the reducer 232 are arranged on the cleaning tray partition plate 221, and the rotating shaft 2321 of the turntable motor and reducer 232 extends into the upper barrel body space 222 to be connected with the turntable 231. In the prior art, the turntable is driven by belt transmission. In this case, the transmission mechanism will be complex and occupy a large space. The present embodiment eliminates the belt transmission mechanism by directly connecting the turntable motor integrated with the reducer to the turntable, so as to simplify the structure of the transmission mechanism and reduce space usage.

A plurality of cup holes 32 for placing reaction cups are arranged on the turntable 231 along the circumferential direction, the top of the cleaning tray barrel body 22 is provided with a reaction cup through hole 33, the reaction cup through hole 33 is used for picking and placing the reaction cups 25, and the reaction cup through hole 33 is at the zero station. When one cup hole 32 in the turntable 231 is rotated to coincide with the central axis of the reaction cup through hole 33, one reaction cup 25 is placed in the cup hole 32 through the reaction cup through hole 33, or the reaction cup 25 in the cup hole 32 is removed through the reaction cup through hole 33. Each cup hole 32 corresponds to one cleaning barrel. When the injection needles and the aspirating needles are cleaned, after the reaction cup 25 is removed from the cup hole 32, and the injection needles and the aspirating needles are moved down to be inserted into the cleaning tank 26 by passing through the cup hole 32 in the turntable for cleaning.

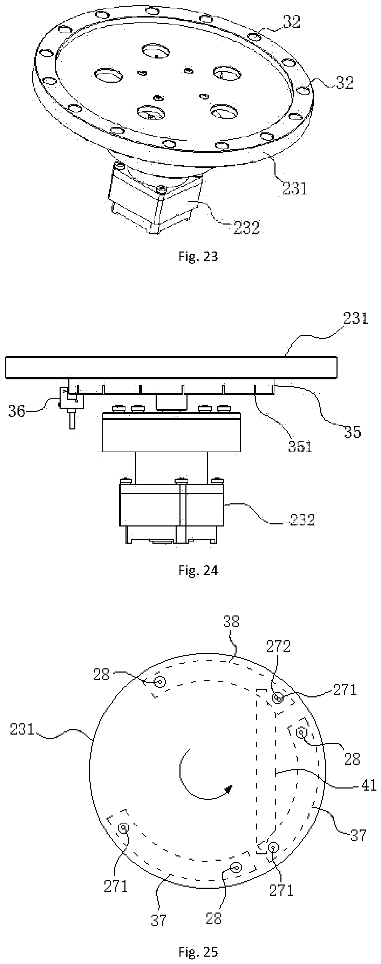

As shown in Fig. 11, the side of the cleaning tray barrel body 22 is also provided with a sensor I 34, and the sensor I 34 is used to detect the number of reaction cups on the turntable and the presence or absence of reaction cups on the turntable. As shown in Fig. 24, the bottom of the turntable 231 is provided with an induction ring 35, the induction ring 35 is provided with a plurality of induction channels 351, a sensor II 36 is also arranged on the cleaning tray partition plate 221 and at the induction ring 35, and when the turntable is rotated, the induction channels 351 on the induction ring 35 are induced by the sensor II 36 to control the rotation angle and position of the turntable.

As shown in Fig. 25, the step of injecting a solution into the reaction cups and the step of aspirating the solution from the reaction cups are set to be performed in one basic cleaning unit, the basic cleaning unit comprises the basic cleaning unit I 37 and the basic cleaning unit II 38, the basic cleaning unit II 38 is a basic cleaning unit before testing, and the basic cleaning unit I 37 is another basic cleaning unit. The basic cleaning unit I 37 and the basic cleaning unit II 38 are successively distributed above the turntable 231 along the circumferential direction of the turntable 231.

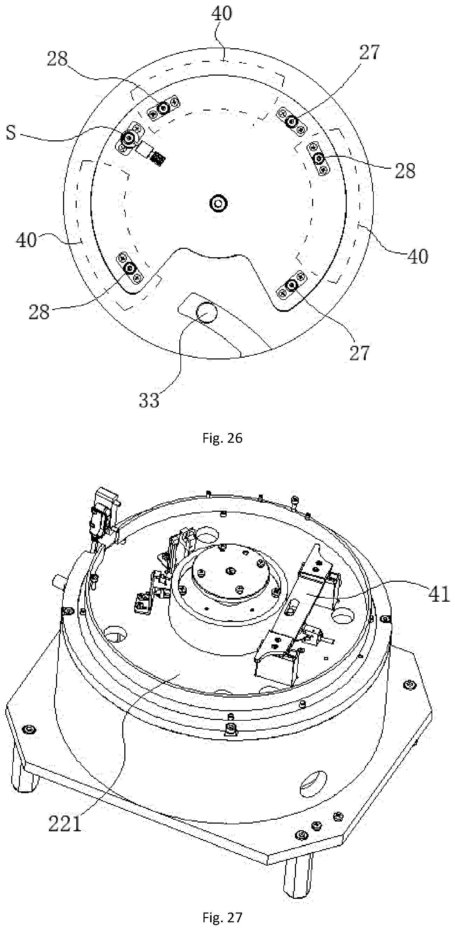

As shown in Fig. 22 and Fig. 26, a magnet retaining ring 39 is arranged on the inner circumferential surface of the cleaning tray barrel body 22 and at the turntable 231. A magnet is arranged on the magnet retaining ring 39 so that magnetic fields 40 are formed in the cleaning tray barrel body 22 and between the injection station and the aspirating station in each basic cleaning unit, the interior of the cleaning tray barrel body 22 at the injection station is located outside the magnetic field 40, and the interior of the cleaning tray barrel body 22 at the aspirating station is located inside the magnetic field 40. After the cleaning solution is injected into the reaction cups at the injection station, the reaction cups enter the magnetic fields 40 with the rotation of the turntable, and the magnetic fields 40 are used to adsorb the magnetic beads in the reaction cups until rotating to the aspirating station. When the solution is aspirated at the aspirating station, the magnetic fields 40 are used to adsorb the magnetic beads in the reaction cups, which can prevent the magnetic beads from being aspirated by the aspirating needles. After aspiration, the reaction cups are removed from the magnetic fields 40 to the injection station where no magnetic field exists, and then the injection needles are used to inject the solution into the reaction cups to impact the magnetic beads, so as to achieve the purpose of cleaning. In the present embodiment, a total of three magnetic fields 40 are formed, the magnet can be a permanent magnet or an electromagnet.

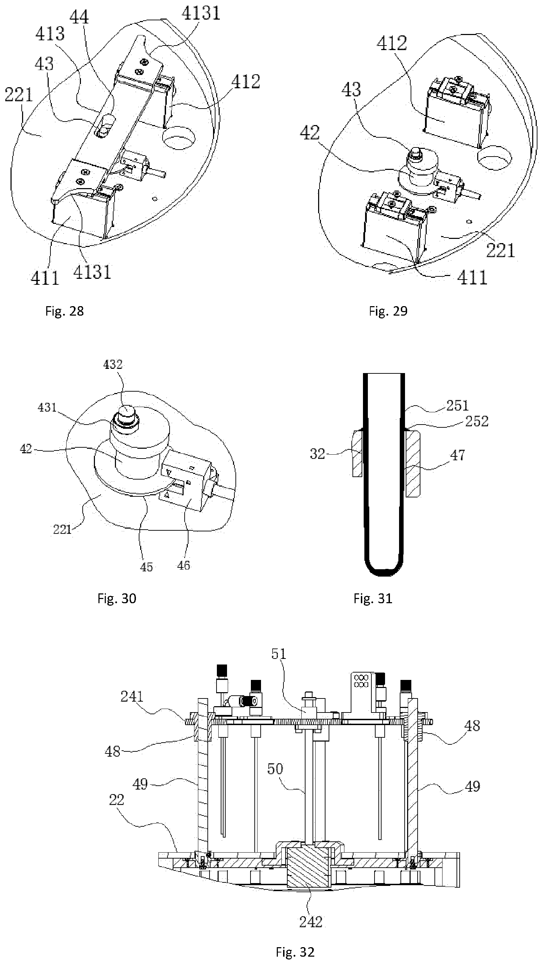

As shown in Fig. 27 and Fig. 28, the cleaning tray of the present embodiment comprises a reaction cup vibrating mechanism 41, and the reaction cup vibrating mechanism 41 is arranged on the cleaning tray partition plate 221 and also located in the upper barrel body space 222. The reaction cup vibrating mechanism 41 comprises a guide rail 1411 and a guide rail II 412 which are arranged on the cleaning tray partition plate 221 and an impactor 413 which is in matched sliding connection to the guide rail 1411 and the guide rail II 412 through a sliding block, an impactor driving mechanism is also arranged on the cleaning tray partition plate 221, and the impactor driving mechanism is used to drive a push block to move back and forth along the guide rail 1411 and the guide rail II 412 so as to use the impactor 413 to repeatedly impact the side of the reaction cups in the cup holes 32 in the turntable located at the injection station so that the magnetic beads in the reaction cups are dispersed by the impact. When the cleaning solution is aspirated from the reaction cups by the aspirating needles and transferred to the injection needles, the magnetic beads are adsorbed together. In the present embodiment, when the injection needles inject the solution into the reaction cups, the reaction cup vibrating mechanism is used to repeatedly impact the reaction cups so that the magnetic beads in the reaction cups are dispersed by the repeated impact, and the magnetic beads are cleaned with the cleaning solution injected by the injection needles, thus ensuring the cleaning effect.

As shown in Fig. 28 and Fig. 29, the impactor driving mechanism comprises an impactor driving motor (not shown in the figures) arranged at the bottom of the cleaning tray partition plate 221 and an impactor rotating shaft I 42 rotationally connected to the top of the cleaning tray partition plate 221, the output shaft of the impactor driving motor is in matched rotational connection with one end of the impactor rotating shaft 142, the end surface of the other end of the impactor rotating shaft 142 is also provided with an impactor rotating shaft II 43, and the central axis of the impactor rotating shaft 142 does not coincide with that of the impactor rotating shaft II 43, i.e., the central axis of the impactor rotating shaft II 43 is in a position that deviates from the central axis of the impactor rotating shaft I 42. The impactor 413 is provided with a slotted hole 44, the impactor rotating shaft II 43 is inserted into the slotted hole 44 for matched sliding connection so as to control the impactor driving motor to drive the impactor rotating shaft 142 and the impactor rotating shaft II 43 to rotate, and the impactor 413 can be driven by the contact between the impactor rotating shaft II 43 and the inner circumferential surface of the slotted hole 44 to move back and forth along the guide rail 1411 and the guild rail II 412 to repeatedly impact the reaction cups.

As shown in Fig. 30, the impactor rotating shaft II 43 comprises an impact bearing 431 and a lock bolt 432, and the impact bearing 431 is locked and fixed on the end surface of the impactor rotating shaft I 42 by the lock bolt 432 by passing through the inner ring of the impact bearing 431. When the impactor rotating shaft II 43 is inserted into the slotted hole 44, the outer ring of the impact bearing 431 is in contact with the inner circumferential surface of the slotted hole 44 for matched sliding connection. Such design can further ensure the smoothness of the impactor during repeated movement. The outer circumferential surface of the impactor rotating shaft I 42 is provided with an induction disk 45, a sensor III 46 is arranged on the cleaning tray partition plate 221 and at the induction disk 45, and the state of the impactor rotating shaft I 42 can be tested by matching the induction disk 45 and the sensor III 46.

As shown in Fig. 25 and Fig. 28, the end of the impactor 413 is set to have an arc shape, and the arc end 4131 is used to impact the sides of the reaction cups 25 so that the reaction cups will not be damaged by the impact.

The reaction cup vibrating mechanism is arranged between the basic cleaning unit II 38 and the basic cleaning unit I 37 adjacent to the basic cleaning unit II, and both arc ends 4131 of the impactor 413 respectively impact a reaction cup located at the injection station in the basic cleaning unit II 38 and a reaction cup located at the injection station in the basic cleaning unit 137. In this way, the reaction cups can be impacted when the solution is injected twice, so as to achieve better impact and cleaning operation, improving the cleaning effect. In the present embodiment, no reaction cup vibrating mechanism is arranged at the first basic cleaning unit I 37, because the reaction cups are placed on the turntable and not or less affected by the magnetic fields before being moved to the injection station of the first basic cleaning unit 137, the adsorption force between the magnetic beads is weak, and after the reaction cups are moved to the injection station of the first basic cleaning unit I 37, the magnetic beads can be dispersed by the cleaning solution, so it is not necessary to conduct vibratory impact.

As shown in Fig. 31, each reaction cup 25 comprises a cup body 251 and a cup body flange 252 arranged on the outer circumferential surface of the cup body 251. After the reaction cup 25 is placed in the cup hole 32 in the turntable 231, an impact gap 47 is left between the cup body 251 located in the cup hole 32 and the inner circumferential surface of the cup hole 32. Such arrangement facilitates the impactor to impact the reaction cup, which further improves the cleaning effect.

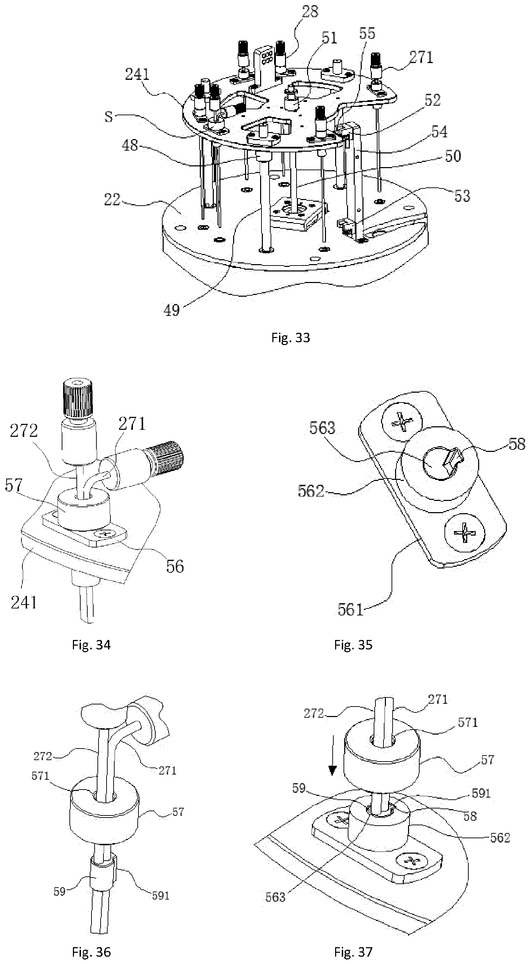

As shown in Fig. 32 and Fig. 33, the needle body lifting mechanism 24 comprises a lifting plate 241 and a lifting motor 242, the lifting plate 241 is provided with a guide sleeve 48, the top surface of the cleaning tray barrel body 22 is provided with a guide rod 49, and the guide rod 49 is in matched connection with the guide sleeve 48 so that the lifting plate 241 is slidably connected to the guide rod 49; and the lifting motor 242 is arranged on the cleaning tray barrel body 22, the output shaft of the lifting motor 242 is in transmission connection with the lead screw 50, the lifting plate 241 is provided with a nut sleeve 51, the lead screw 50 is matched with the nut sleeve 51 to form a lead screw nut mechanism so that the lifting plate 241 can be controlled by the action of the lifting motor 242 to move up and down, and the injection needles and the aspirating needles are arranged on the lifting plate 241. In addition, the bottom openings of the injection needles are tilted so that the cleaning solution sprayed through the bottom openings of the injection needles can be flushed onto the reaction cups, because the reaction cups apply a magnetic field at the aspirating station, the magnetic beads are adsorbed on the inside walls of the reaction cups. After the magnetic field disappears, part of the magnetic beads are adsorbed on the inside walls of the reaction cups. The bottom openings of the injection needles are tilted to further ensure that the cleaning solution can disperse the magnetic beads adsorbed on the inside walls of the reaction cups, so as to further ensure the cleaning effect. The bottom side walls of the aspirating needles near the bottom openings of the aspirating needles are provided with notches to prevent the bottom openings of the aspirating needles from being blocked when the aspirating needles are inserted into the bottoms of the reaction cups, ensuring the normal aspirating operation.

The top surface of the cleaning tray barrel body 22 is also provided with an upper position sensor 52 and a lower position sensor 53, and the upper position sensor 52 and the lower position sensor 53 are arranged on the top surface of the cleaning tray barrel body 22 through support rods 54. The lifting plate 241 is also provided with an induction sheet 55, and the upper limit position and the lower limit position of the lifting plate 241 can be detected by matching the induction sheet 55 with the upper position sensor 52 and the lower position sensor 53. In the working process, when the lifting plate 241 is moved up to the upper limit position, the injection needles and the aspirating needles on the lifting plate 241 have been moved up into place and have been removed from the reaction cups. At this time, the turntable can drive the reaction cups to rotate. When the lifting plate 241 is moved down to the lower limit position, the aspirating needles on the lifting plate 241 have been inserted into the levels of the reaction cups, and the aspirating operation is ready.

As shown in Fig. 34 and Fig. 35, the injection needle 1271 and the injection needle II 272 are connected to the lifting plate 241 through a limiting block, the limiting block comprises a base 56 fastened on the lifting plate 241 through a screw and a screw cap 57 threaded to the base 56, the base 57 comprises a bottom plate 561 and a cylinder body 562 arranged on the bottom plate 561, the cylinder body 562 and the bottom plate 561 are connected through a mounting through hole 563, the inner circumferential surface of the cylinder body 562 penetrated by the mounting through hole 563 is provided with a groove 58, the groove 58 is concave along the radial direction of the cylinder body 562, one side of the groove 58 is open, and the other three sides are closed.

As shown in Fig. 36 and Fig. 37, the bottom of the screw cap 57 is provided with a screw cap through hole 571, the injection needle I 271 and the injection needle II 272 are provided with a guide block 59 respectively, the guide block 59 is cylindrical, the diameter thereof is matched with that of the mounting through hole 563, and one side of the guide block 59 is provided with a bump 591 protruding radially. During installation, the injection needle 1271 and the injection needle II 272 are successively penetrated through the screw cap through hole 571 of the screw cap 57, the guide block 59 and the mounting through hole 563 of the base 56 so that the bump 591 of the guide block 59 is stuck into the groove 58, the groove 58 is matched with the bump 591 to limit the injection needle I 271 and the injection needle II 272 in the circumferential direction, the screw cap 57 is tightened onto the cylinder body 562, and the bump 591 is pressed into the groove 58 by the screw cap 57 to limit the injection needle 1271 and the injection needle II 272 in the axial direction. With the above structural design, the injection needle I 271 and the injection needle II 272 are connected to the lifting plate 241, and the positions of the injection needle 1271 and the injection needle II 272 are fixed, ensuring the normal injection operation.

As shown in Fig. 25, during cleaning in the present embodiment, the turntable 231 drives the reaction cups to rotate so that the reaction cups are successively passed through the basic cleaning unit I 37 and the basic cleaning unit II 38 to complete the cleaning. Supposing that the number of rotation turns of a reaction cup driven by the turntable 231 is set to N2, N2≥2.

When N2=2, the cleaning steps comprise S1 cleaning step for the first turn and S2 cleaning step for the second turn, wherein the S1 cleaning step for the first turn comprises:
1). Controlling the turntable 231 to drive the reaction cup (not shown in the figure) to rotate to the injection station in the first basic cleaning unit 137, and stopping rotation;
2) Controlling the injection needle I 271 located at the injection station in the first basic cleaning unit I 37 to move down to be inserted into the reaction cup to inject a cleaning solution A into the reaction cup, and cleaning magnetic beads in the reaction cup with the injected cleaning solution A; and after injection, controlling the injection needle 1271 at the injection station in the first basic cleaning unit I 37 to move up to the original position;
3). Controlling the turntable 231 to drive the reaction cup to rotate to the aspirating station in the first basic cleaning unit 137, and stopping rotation;
4) Controlling the aspirating needle 28 located at the aspirating station in the first basic cleaning unit I 37 to move down to be inserted into the reaction cup to aspirate the cleaning solution from the reaction cup, and draining the cleaning solution; and after drainage, controlling the aspirating needle 28 at the aspirating station in the first basic cleaning unit I 37 to move up to the original position;
5). Controlling the turntable 231 to drive the reaction cup to rotate to the injection station in the second basic cleaning unit 137, and stopping rotation;
6) Controlling the injection needle 1271 located at the injection station in the second basic cleaning unit I 37 to move down to be inserted into the reaction cup to inject the cleaning solution A into the reaction cup, controlling the reaction cup vibrating mechanism to impact the reaction cup, and cleaning the magnetic beads in the reaction cup with the injected cleaning solution A and the impact; and after injection, controlling the injection needle 1271 at the injection station in the second basic cleaning unit I 37 to move up to the original position;
7). Controlling the turntable 231 to drive the reaction cup (not shown in the figure) to rotate to the aspirating station in the second basic cleaning unit 137, and stopping rotation;
8) Controlling the aspirating needle 28 located at the aspirating station in the second basic cleaning unit I 37 to move down to be inserted into the reaction cup to aspirate the cleaning solution from the reaction cup, and draining the cleaning solution; and after drainage, controlling the aspirating needle 28 at the aspirating station in the second basic cleaning unit I 37 to move up to the original position;
9). Controlling the turntable 231 to drive the reaction cup (not shown in the figure) to rotate to the injection station in the basic cleaning unit II 38, and stopping rotation;
10) Controlling the injection needle 1271 located at the injection station in the basic cleaning unit II 38 to move down to be inserted into the reaction cup to inject the cleaning solution A into the reaction cup, controlling the reaction cup vibrating mechanism to impact the reaction cup, and cleaning the magnetic beads in the reaction cup with the injected cleaning solution A and the impact; and after injection, controlling the injection needle 1271 at the injection station in the basic cleaning unit II 38 to move up to the original position;
11). Controlling the turntable 231 to drive the reaction cup (not shown in the figure) to rotate to the aspirating station in the basic cleaning unit II 38, and stopping rotation;
12) Controlling the aspirating needle 28 located at the aspirating station in the basic cleaning unit II 38 to move down to be inserted into the reaction cup to aspirate the cleaning solution from the reaction cup, and draining the cleaning solution; and after drainage, controlling the aspirating needle 28 at the aspirating station in the second basic cleaning unit I 37 to move up to the original position;

The S1 cleaning step for the first turn is completed through the above steps.

The difference between the S2 cleaning step for the second turn and the S1 cleaning step for the first turn is step 10), and the other steps are the same as those in the S1 cleaning step for the first turn, that is:

10) Controlling the injection needle II 272 located at the injection station in the basic cleaning unit II 38 to move down to be inserted into the reaction cup to inject a cleaning solution B into the reaction cup, controlling the reaction cup vibrating mechanism to impact the reaction cup, and cleaning the magnetic beads in the reaction cup with the injected cleaning solution B and the impact; and after injection, controlling the injection needle II 272 at the injection station in the basic cleaning unit II 38 to move up to the original position;

When N2>2, the cleaning steps comprises T1 cleaning step for other turns and T2 cleaning step for the last turn, the T1 cleaning step for other turns is the same as the S1 cleaning step for the first turn, and the T2 cleaning step for the last turn is the same as the S2 cleaning step for the second turn.

In the present embodiment, two cleaning solutions are used in the whole cleaning process: the cleaning solution A and the cleaning solution B. The reaction cup is injected with the cleaning solution A in the injection step of the basic cleaning unit I 37, and the reaction cup is injected with the cleaning solution B in the injection step of the basic cleaning unit II 38. The cleaning solution A has strong cleaning ability with more residual bubbles after cleaning, and the cleaning solution B has no residual bubbles. After entering the basic cleaning unit I 37, the injected cleaning solution A is used to clean the magnetic beads in the reaction cup. After entering the basic cleaning unit II 38, the injected cleaning solution B is used to remove residual bubbles in the reaction cup.

The two different cleaning solutions are used to cooperate with each other, which can not only achieve the effect of cleaning the magnetic beads in the reaction cup, but also remove the residual bubbles in the reaction cup.

"A plurality of" in the present embodiment means a quantity of "two or more than two". The above embodiments are merely used for illustration of the present invention, and not intended to limit the present invention. Various changes or transformations can also be made by those skilled in the art without departing from the spirit and the scope of the present invention. Therefore, all equivalent technical solutions shall also belong to the protection scope of the present invention, and the protection scope of the present invention shall be defined by the claims.

## Claims

1. A method for mixing immunoassay reagent in immunoassay analysis device, **characterized in that**: comprising
a process of dispersing aggregated magnetic beads in a reagent tray of an immunoassay analysis device by keeping an immunoassay reagent tube containing the immunoassay reagent in a variable speed state during rotation and by exerting an impact force on the immunoassay reagent tube during rotation of the immunoassay reagent tube, so as to keep the immunoassay reagent in a mixed state, wherein the process further comprising following steps, wherein
continuous teeth distributed on a toothed disc (5) in the reagent tray along a circumferential direction are designed into discontinuous teeth so that a plurality of tooth segments (511) and notch segments (512) are formed on the toothed disc (5), a kit (1) is installed on a reagent turntable (4), the immunoassay reagent tube is rotationally connected to the kit (1), and the reagent turntable (4) rotates relative to the toothed disc (5), so as to drive a reagent tube gear (311) at a bottom of the immunoassay reagent tube to move along the circumferential direction of the toothed disc (5);
in a working process, when the immunoassay reagent tube (3) is located on the tooth segments (511) on the toothed disc (5), the tooth segments (511) are engaged with the reagent tube gear (311) at the bottom of the immunoassay reagent tube (3) to make the immunoassay reagent tube (3) rotate; when the immunoassay reagent tube (3) is located on the notch segments (512) on the toothed disc (5), the immunoassay reagent tube (3) loses power of rotation so that a rotation speed of the immunoassay reagent tube (3) in the notch segments (512) is changed once; and when the immunoassay reagent tube (3) is located on the tooth segments (511) on the toothed disc (5) again, the tooth segments (511) is engaged with the reagent tube gear (311) at the bottom of the immunoassay reagent tube (3) again, and the rotation speed of the immunoassay reagent tube (3) is changed again to return to a rotation state, so repeatedly, until the rotation speed of the immunoassay reagent tube (3) is repeatedly in the variable speed state in the working process;
when the immunoassay reagent tube (3) is moved from the notch segments (512) to the tooth segments (511) on the toothed disc (5), the tooth segments (511) that are just engaged with the reagent tube gear (311) form the impact force on the immunoassay reagent tube (3), which plays an impact role.

2. The method for mixing immunoassay reagent in immunoassay analysis device according to claim 1, **characterized in that**: an arc length of the tooth segments (511) is set to L, and an arc length of the notch segments (512) is L/2.

3. The method for mixing immunoassay reagent in immunoassay analysis device according to claim 1, **characterized in that**: an inside wall of the immunoassay reagent tube (3) is provided with tube bumps (312); and the immunoassay reagent tube (3) further mixs the immunoassay reagent by the tube bumps (312) during rotation at the variable speed and under the action of the impact force.

4. The method for mixing immunoassay reagent in immunoassay analysis device according to claim 3, **characterized in that**: the tube bumps (312) comprise two tube bumps (312), and the two tube bumps (312) are distributed symmetrically about a central axis of the immunoassay reagent tube (3).

5. The method for mixing immunoassay reagent in immunoassay analysis device according to claim 1, **characterized in that**: in the working process, when the reagent is not aspirated, the reagent turntable (4) is held still by controlling the rotation of the toothed disc (5) so that the immunoassay reagent tube (3) rotates;
when the reagent is aspirated in the kit, the reagent turntable (4) is controlled to drive the kit to rotate to a reagent aspirating point A, the kit at the reagent aspirating point A is held still, and then the reagent in the kit is aspirated.

6. The method for mixing immunoassay reagent in immunoassay analysis device according to claim 5, **characterized in that**: a rotation direction of the toothed disc (5) is controlled to be opposite to that of the reagent turntable (4).

7. An immunoassay analysis device, **characterized in that**: comprising
A reagent tray (6); and
a cleaning tray (16) arranged on one side of the reagent tray (6), wherein the reagent tray (6) adopts the method for mixing immunoassay reagent in immunoassay analysis device according to any of claim 1 to claim 6 to mix an immunoassay reagent.

8. The immunoassay analysis device according to claim 7, **characterized in that**: the cleaning tray (16) comprises a cleaning tray barrel body (22), a turntable mechanism (23) arranged in the cleaning tray barrel body (22) and a needle body lifting mechanism (24) arranged above the cleaning tray barrel body (22), injection needles (27) and aspirating needles (28) are arranged on the needle body lifting mechanism (24), reaction cups (25) are placed on a turntable (231) of the turntable mechanism (23), the reaction cups (25) are driven through the turntable (231) to rotate, a cleaning tank (26) is arranged in the cleaning tray barrel body (22) and below the turntable (231), and the injection needles (27) and the aspirating needles (28) are driven by a downward movement of the needle body lifting mechanism (24) to move down to be inserted into the reaction cups (25) to clean the reaction cups (25) and to move down to be inserted into the cleaning tank (26) to be cleaned after the reaction cups (25) are removed.

9. The immunoassay analysis device according to claim 8, **characterized in that**: the cleaning tank (26) comprises a fully enclosed annular tank body (261), and injection needle cleaning barrels (262) and aspirating needle cleaning barrels (263) which are arranged on the annular tank body (261), wherein an inner space of the injection needle cleaning barrels (262) and the aspirating needle cleaning barrels (263) is communicated with an inner space of the annular tank body (261), a bottom surface of the annular tank body (261) is also provided with a drain pipe (264) for draining water, and top surfaces of each of the injection needle cleaning barrels (262) and each of the aspirating needle cleaning barrels (263) are respectively provided with openings for the injection needles (27) and the aspirating needles (28) to insert; and the injection needle cleaning barrels (262) and the aspirating needle cleaning barrels (263) are arranged according to positions of the injection needles (27) and the aspirating needles (28) so that one of the injection needles (27) is cleaned by one of the injection needle cleaning barrels (262), and one of the aspirating needles (28) is cleaned by one of the aspirating needle cleaning barrels (263).

10. The immunoassay analysis device according to claim 9, **characterized in that**: each of the aspirating needle cleaning barrels (263) is vertically penetrated through the annular tank body (261), each of the aspirating needle cleaning barrels (263) comprises an outer barrel body (2631) and an inner barrel body (2632) arranged in the outer barrel body (2631), an annular space is formed between the outer barrel body (2631) and the inner barrel body (2632), the annular space is communicated with the inner space of the annular tank body (261), a top surface of the inner barrel body (2632) is lower than that of the outer barrel body (2631), the opening (29) of each of the aspirating needle cleaning barrels (263) is arranged on a top surface of the outer barrel body (2631), the aspirating needle cleaning barrel arranged below the annular tank body (261) is also provided with a water inlet (31), the water inlet (31) is communicated with a bottom of an inner cavity (26321) of the inner barrel body (2632), and a top of the inner cavity (26321) of the inner barrel body (2632) is communicated with an inner space of the outer barrel body (2631).

11. The immunoassay analysis device according to claim 9, **characterized in that**: each of the injection needle cleaning barrels (262) comprises a barrel body (2621), a bottom end of the barrel body (2621) is arranged at a top of the annular tank body (261), an inner cavity (26321) of the barrel body (2621) is communicated with the inner space of the annular tank body (261), and the opening (29) of each of the injection needle cleaning barrels (262) is arranged on a top end of the barrel body (2621).

12. The immunoassay analysis device according to claim 9, **characterized in that**: a reaction cup vibrating mechanism (41) is arranged in the cleaning tray barrel body (22), the reaction cup vibrating mechanism (41) comprises a guide rail I (411) and a guide rail II (412) which are arranged in the cleaning tray barrel body (22) and an impactor (413) which is slidably connected to the guide rail I (411) and the guide rail II (412), an impactor driving mechanism is also arranged in the cleaning tray barrel body (22), and the impactor driving mechanism is used to drive a push block to move back and forth along the guide rail I (411) and the guide rail II (412) so as to use the impactor (413) to repeatedly impact the reaction cups (25) on the turntable located at an injection station so that the magnetic beads in the reaction cups (25) are dispersed by the impact.

13. The immunoassay analysis device according to claim 12, **characterized in that**: the impactor driving mechanism comprises an impactor driving motor arranged in the cleaning tray barrel body (22) and an impactor rotating shaft I (42) rotationally connected in the cleaning tray barrel body (22), an output shaft of the impactor driving motor is in matched rotational connection with one end of the impactor rotating shaft I (42), an end surface of an other end of the impactor rotating shaft I (42) is also provided with an impactor rotating shaft II (43), a central axis of the impactor rotating shaft I (42) does not coincide with that of the impactor rotating shaft II (43), the impactor (413) is provided with a slotted hole, the impactor rotating shaft II (43) is inserted into the slotted hole for matched sliding connection so as to control the impactor driving motor to drive the impactor rotating shaft I (42) and the impactor rotating shaft II (43) to rotate, and the impactor (413) is driven by the contact between the impactor rotating shaft II (43) and an inner circumferential surface of the slotted hole to move back and forth along the guide rail I (411) and the guild rail II to repeatedly impact the reaction cups (25).

14. The immunoassay analysis device according to claim 8, **characterized in that**: three basic cleaning units are successively arranged on the needle body lifting mechanism (24) along a circumferential direction of the turntable (231), the basic cleaning units comprise two basic cleaning units I and one basic cleaning unit II, an injection needle I (271) and an aspirating needle (28) are arranged in each of the basic cleaning units I, an integrated double injection needles (S) and an aspirating needle (28) are arranged in the basic cleaning unit II, and the integrated double injection needles (S) comprises another injection needle I (271) and an injection needle II (272); and along the circumferential direction of the turntable (231), the injection needle I (271) and the aspirating needle (28) of one of the basic cleaning units I, the injection needle I (271) and the aspirating needle (28) of another one of the basic cleaning units I, the integrated double injection needles (S) and the another one aspirating needle (28) are distributed on the needle body lifting mechanism (24) in order.

15. The immunoassay analysis device according to claim 14, **characterized in that**: the integrated double injection needles (S) connected to a lifting plate (241) of the needle body lifting mechanism (24) through a limiting block, the limiting block comprises a base (56) arranged on the lifting plate (241) and a screw cap (57) threaded to the base (56), the base (56) comprises a bottom plate (561) and a cylinder body (562) arranged on the bottom plate (561), the cylinder body (562) and the bottom plate (561) are connected through a mounting through hole (563), an inner circumferential surface of the cylinder body (562) penetrated by the mounting through hole (563) is provided with a groove (58), the groove (58) is concave along a radial direction of the cylinder body (562), one side of the groove (58) is open, and other three sides of the groove (58) are closed;
a bottom of the screw cap (57) is provided with a screw cap through hole (571), the integrated double injection needles (S) also provided with a guide block (59) which is cylindrical, a diameter of the guide block (59) is matched with that of the mounting through hole (563), and one side of the guide block (59) is provided with a bump (591) protruding radially; and during installation, the injection needle I (271) and the injection needle II (272) are successively penetrated through the screw cap through hole (571) of the screw cap (57), the guide block (59) and the mounting through hole (563) of the base (56) so that the bump (591) of the guide block (59) is stuck into the groove (58), the groove (58) is matched with the bump (591) to limit the injection needle I (271) and the injection needle II (272) in the circumferential direction, the screw cap (57) is tightened onto the cylinder body (562), and the bump (591) is pressed into the groove (58) by the screw cap (57) to limit the injection needle I (271) and the injection needle II (272) in the axial direction.
